# DEMANDE DE BREVET EUROPEEN

(11) **EP 1 707 212 A1**
(43) Date de publication de la demande: **04.10.2006**
(21) Numéro de dépôt: 05290680.7
(22) Date de dépôt: 25.03.2005
(51) Int. Cl.: A61K 36/534

(54) **Menthe pouillot pour le traitement de l'infection par le VIH**

(71) Demandeur: Martin- Jacqueline, 84210 Pernes-Les-Fontaines (FR)
(72) Inventeur: Martin- Jacqueline, 84210 Pernes-Les-Fontaines (FR)

(57) **Abrégé**

La menthe pouillot est utilisée pour la fabrication d'un médicament pour le traitement de l'infection par le VIH et permet la guérison de patients atteints par le SIDA.

## Description

Le VIH se présente sous la forme d'une particule sphérique,le virion enveloppé par une double couche de lipides empruntés à la membrane de la cellule qu'il infecte. IL contient aussi les deux brins d'ARN constituant le génome viral. Il emprisonne les cellules du malade atteint, le rend prisonnier, incapable de se débarasser de ce virus. Au contraire ce virus se multiplie très très vite et enkylose toutes les parties du corps du malade aux fils des années. Le malade va devenir paralysé, dépendant à 100% du VIH.

La menthe Pouillot est un produit qui a une forte odeur désagréable et très puissante. Sous huit jours de prise de ce médicament, le malade se libère de l'emprise du VIH total qui est en lui. Le VIH abadonne le malade. Le malade redevient apte à gérer ses mouvements, reprend le goût de vivre, il redevient autonome, il retrouve à 100% sa santé après 21 jours de traitement à la menthe Pouillot ( Exemple: une malade atteinte depuis 20 ans peut remarcher pendant deux heures de suite, alors qu'elle était sur un fauteul roulant).

Trois gouttes de menthe Pouillot par jour sont faciles à prendre, surtout lorsque je vois le résultat que ça donne.

## Revendications

1. En prévision de guérir les gens atteints du virus du sida, je cite la menthe Pouillot dont j'ai moi-même trouver les effets de guérison à 100% du VIH et dont je fais un test sur une personne atteinte du sida depuis vingt ans. Ce sujet disposait d'énormes séquelles sur un fauteuil roulant, de gros problèmes dans son cerveau. Cette malade aura fini son traitement le 15 Avril 2005 et fera une analyse biologique pour vérifier mon résultat.
Je réclame mon droit personnel unique à utiliser la menthe Pouillot sur des malades atteints du sida en vue de les guérir. Je suis la seule à avoir trouvé ce procédé de guérison, moi seule en détient le droit de l'utiliser.
